# EUROPEAN PATENT SPECIFICATION

(11) **EP 4 171 405 B1**
(45) Date of publication and mention of the grant of the patent: **11.09.2024**
(21) Application number: 21758419.2
(22) Date of filing: 17.06.2021
(51) Int. Cl.: A61B 17/34, A61B 5/00, A61B 34/20

(54) **INTRAOSSEOUS IMPLANTABLE MICROSENSORS**
INTRAOSSALE IMPLANTIERBARE MIKROSENSOREN
MICRO-CAPTEURS IMPLANTABLES DANS UN OS

(30) Priority: 26.06.2020 US 202063044665 P
(43) Date of publication of application: 03.05.2023
(73) Proprietor: Prometheus Regeneration R&D Limited, Glasgow G12 9DA (GB)
(72) Inventor: PICARD, Guillaume, Newark, DE 19711 (US); PICARD, Ludovic, Glasgow G77 5QN (GB); PICARD, Frederic, Glasgow G77 5QN (GB); MARTIN, Philippe P., 75014 Paris (FR)
(74) Representative: Samson & Partner Patentanwälte mbB
(86) International application number: PCT/IB2021/000417
(87) International publication number: WO 2021/260428

(56) References cited:
- EP-A1- 2 929 836
- EP-A2- 1 328 203
- EP-B1- 1 328 203
- CA-A1- 2 458 966
- DE-A1- 102005 014 624
- US-A1- 2007 179 739
- US-A1- 2009 048 575
- US-A1- 2009 187 120
- US-A1- 2009 312 782
- US-A1- 2010 191 100
- US-A1- 2013 338 455

## Description

### Field

The present invention relates to devices for implantation of biosensors and, in particular, microsensors suitable for intraosseous implantation to track the kinematics of a subject, especially movement and/or orientation of a subject's bones and/or prostheses.

### Background

Motion tracking is of increasing interest for numerous applications, such as orthopaedics and sports medicine. While orthopaedic surgery is sometimes guided by navigational systems, post-surgical biosensors typically are not employed. Additionally, clinical or surgical navigation systems are often large instruments confined to a single office or surgical theater and rely upon multiple video cameras and complex imaging software to detect and analysis motion.

Wearable inertial systems have also been proposed, particularly for sports injury rehabilitation, but that they are typically not fixed to the subject's bone or prothesis and thus suffer from the disadvantage of data degradation due to indirect fixation and soft tissue artifacts.

Patent documents DE102005014624 A1, EP1328203 A2 and US2009/048575 A1 disclose devices for drilling a channel into bone tissue and deliver an implant to the bone channel.

### Summary

The invention is defined by independent claim 1. Further embodiments are defined by the dependent claims. In this disclosure implantable biosensors and methods of making and using such biosensors are disclosed. The biosensors can be micro-devices, for example, micro-sized bead implants having an associated gyroscope, accelerometer and/or magnetometer to detect and transmit changes in the position of the biosensor following implantation. The biosensors can be implanted into a subject's bone and/or a subject's prosthesis to detect, for example, changes in position or orientation of a prosthetic implant that can indicate loosening or potential onset of structural failures.

In one aspect of the invention, hollow microbead devices are disclosed for intraosseous implantation, e.g., within a cortical bone segment of interest in a subject's body. The devices can comprise a hard shell that can withstand implantation and internal electronics to communicate the device's location. For example, the device can comprise a shell for enclosing a biosensor and be configured for passage through the lumen of a trocar for implantation into a bone segment. The biosensor can include at least one kinematic sensor disposed within the shell, e.g., at least one sensor from the group of accelerometers, gyroscopes and magnetic sensors.

The biosensors of the present disclosure can be constructed as microelectromechanical systems (MEMS). In one embodiment, the biosensor can be a graphene-based MEMS device. The biosensors can be encased within a shell, e.g., a titanium, graphene or ceramic shell. The shell can be rounded or at least partially ovaloid in shape (e.g., a microbead) with an equatorial dimension chosen such that the device can pass easily through a cylindrical inner lumen of a trocar for delivery to a target site in a cortical bone segment. For example, the equatorial dimension can be between 2 millimeters and 200 micrometers, preferably in some instances, less than 1 millimeter. In some embodiments, the shell can have a convex anterior surface configured to mate with a concave bottom surface of a cavity formed in a bone segment.

In another aspect of the disclosure, methods of implanting biosensors are disclosed that can include the steps of forming a cavity in a bone segment; loading a biosensor into an implant delivery instrument, aligning a distal end of the delivery instrument with the cavity, and activating the delivery instrument to implant the biosensor into the cavity. The step of forming the cavity can further comprise accessing a bone with a hollow trocar, inserting a drill into the hollow trocar and activating the drill to remove a portion of the bone segment to form the cavity, wherein the cavity is preferably formed in cortical bone. In certain embodiments, the cavity can be cylindrical with a concave bottom and the biosensor can be a microbead with a convex surface that mates to the concave bottom surface of the cavity. The microbeads can be manually implanted or affixed with mechanical or pneumatic assistance.

The step of activating the delivery instrument can further comprise utilizing mechanical force to press the biosensor into the cavity. For example, the biosensor can be a microbead with a diameter that is larger than the width of the cavity and the method can further comprise applying force to secure the biosensor in the cavity by frictional engagement. The method can further include a step of sealing the cavity following implantation, e.g., by applying a sealant on top of the microbead following implantation of the microbead in the cavity. In certain embodiments, the biosensor can comprise a kinematic sensor, e.g., with at least one sensor element selected from the group of accelerometers, gyroscopes and magnetic sensors.

According to the invention, devices for implantation of a biosensor into a bone segment are disclosed as defined in claim 1. In certain embodiments, the trocar is releasable couplable to the instrument body and, optionally, also includes a Luer-lock type coupler for connecting the trocar to the instrument body. The trocar, drill cartridge and biosensor implantation cartridge can all be replaceable and/or disposable.

In certain embodiments, the drill cartridge can further comprise a drill actuator for the drill. The drill can comprise a rotatable shaft with the drill tip disposed at its distal end. The drill actuator can further include a rotary motor or, optionally, a drive coupler for coupling the rotatable shaft to a separate rotary motor. The drill cartridge is preferable designed such that the drill and, optionally, the motor as well, can travel longitudinally to be deployed at a target bone segment site and to bore into the bone segment to form a cavity for a microbead biosensor.

In certain embodiments, the biosensor implantation cartridge can comprise a cylindrical chamber for storing at least one biosensor prior to implantation and an implantation actuator, e.g., a piston alone or a piston together with a pneumatic coupler for coupling the piston to a pneumatic pressure source. The devices of the present invention can further comprise a stop for limiting the penetration of drill, the biosensor(s), or both into the bone segment.

In yet a further aspect of the disclosure, methods for monitoring physiological kinematics are disclosed, which include can comprise the steps of implanting at least one microbead biosensor into a cortical bone segment of a subject, and receiving signals from the microbead biosensor over time to monitor movement of the bone segment. The microbead biosensors can comprise a shell for enclosing a sensor and configured for passage through the lumen of a trocar for implantation into a bone segment, and at least one kinematic sensor disposed within the shell. In certain embodiments, the biosensor is a kinematic sensor further comprises at least one sensor from the group of accelerometers, gyroscopes and magnetic sensors. The methods can further comprise transmitting kinematic data to an external analyzer and/or transmitting kinematic data to an wearable data storage device.

In certain embodiments, the method can further comprise implanting a plurality of kinematic sensors into different bone segments either in the same bone or in separate bones or both, and receiving signals from the microbead biosensors over time to determine movement of one implanted microbead biosensor relative to another implanted microbead biosensor. For example, the method can further comprise tracking kinematic output data for each sensor, such as (1) the locations of the sensors in 3D space or the distances between sensors, (2) movement in 3D space of one sensor relative to another sensor or relative to a fixed frame of reference, (3) acceleration in 3D space of one sensor relative to another sensor or relative to a fixed frame of reference, and/or (4) rotation in 3D space of one sensor relative to another sensor or relative to a fixed frame of reference.

Systems for monitoring physiological kinematics can comprise at least one microbead kinematic biosensor adapted to be implanted into a bone segment, and an external receiver for receiving kinematic signals from the microbead kinematic biosensor. Such a kinematic biosensor can include at least one sensor from the group of accelerometers, gyroscopes and magnetic sensors. The biosensor can further comprise a power source such as a battery and, optionally, an energy harvesting device to recovery energy from movement of the bone segment. The biosensor can include a processor and a memory and optionally the memory comprises a non-volatile memory component. The biosensor can further comprise a transceiver to transmit data to the external receiver and, optionally, a contactless energy coupler adapted to receive energy from a charging unit. The charging unit can be a stand-alone apparatus or part of the external receiver.

The biosensors, methods and systems described herein are applicable to monitoring a wide range of physiological conditions including, for example, guidance or augmentation of prosthetic procedures such as hip, knee or other joint repairs, reconstructions or replacement surgeries, as well as other orthopaedic or sports medicine procedures where it is useful to monitor body structures and to obtain data regarding the kinematics on such structures.

The present invention can be used by physiotherapists, surgeons and radiologists. It can be a useful tool for motion tracking for sports performance or gait analysis, e.g., for surgery, recovery or rehabilitation. Because the biosensors of the present invention can be implanted directly into bone segments, greater accuracy in measuring kinematic parameters can be expected vis-à-vis wearable sensors.

The present disclosure provides device for a micro-sized hollow bead implant comprising: a an external shell; and a circuit in the bead having a power element to provide power to the circuit; at least one sensor contained within the implant comprising (1) an accelerometer to provide a sensor output in response to acceleration of the implant, (2) a gyroscope to provide a sensor output in response to positioning (angular orientation and velocity) of the implant, or (3) a magnetoelectric element to provide a sensor output in response to orientation of the implant in a magnetic field which can be the natural polar magnetic field or one created in the room is used for a control environment, alone or in combination; and a processor to generate a data output in response to the sensor output; and a transceiver to communicate with devices outside of the body. In this embodiment, the bead can be placed within the cortical bone of a segment of interest of a patient's body. The devices can also include energy harvesting component to extract operating power for the subject's own movements. Alternatively or in addition, the devices can include contactless charging technology, e.g., inductive coil energy transfer mechanisms. In this embodiment, the bead can be placed within the cortical bone of a segment of interest. In some embodiments, the bead is implanted using a specially designed tool and implantation method.

In further embodiments, the circuit is an integrated circuit. Preferably the accelerometer, gyroscope or magnetoelectric are provided as microelectromechanical systems (MEMS) devices. In further embodiments, the MEMS device can employ graphene sensor technologies. In some embodiments the power element comprises a battery to actively power the circuit. In preferred embodiments the power element comprises an inductor and regulator for receiving electromagnetic radiation energy so as to passively power the circuit or in combination with a battery or supercapacitor. In some embodiments, the exterior shell is constructed from graphene.

The present disclosure also provides a system for the determination of initial and subsequent motion including orientation of a segment of interest of a patient's body comprising: a micro-sized hollow bead implant placed within the cortical bone of a segment of interest of a patient's body and a circuit in the implant having a battery to provide power to the circuit, at least one sensor contained within the implant comprising (1) an accelerometer to provide a sensor output in response to acceleration of the implant, (2) a gyroscope to provide a sensor output in response to positioning of the implant, or (3) a magnetoelectric element to provide a sensor output in response to positioning of the implant in a magnetic field, alone or in combination, a processor to generate a data output in response to the sensor output, and a transceiver to generate a signal in response to the data output when the circuit is supplied with power; and a remote receiving unit for receiving the data signal generated by the transceiver outside of the patient's body to determine the motion including orientation of the segment of the patient's body. In still further embodiments the circuit is an integrated circuit. Preferably the gyroscope, accelerometer, and/or magnetoelectric are provided as microelectromechanical (MEMS) and/or magnetic fields systems devices.

The systems according to the present disclosure can determine initial and subsequent motion including orientation of a segment of interest of a patient's body comprising: a micro-sized hollow bead implant placed within the cortical bone of a segment of interest of a patient's body and a circuit in the implant having a battery to provide power to the circuit, at least one sensor contained within the implant comprising (1) an accelerometer to provide a sensor output in response to acceleration of the implant, (2) a gyroscope to provide a sensor output in response to positioning of the implant, or (3) a magnetoelectric element to provide a sensor output in response to positioning of the implant in a magnetic field, alone or in combination, a processor to generate a data output in response to the sensor output, and a transceiver to generate a signal in response to the data output when the circuit is supplied with power; and a remote powering/receiving unit for supplying energy to the inductor and receiving the data signal generated by the transceiver outside of the patient's body, wherein when power is supplied to the circuit by the powering/receiver unit the sensor provides a sensor output in response to acceleration or orientation of the implant, and the powering/receiver unit receives the data signal from the transceiver to determine the initial and subsequent motion including orientation of a segment of interest of a patient's body. In still further embodiments the circuit is an integrated circuit. Preferably the accelerometer, gyroscope and/or the magnetoelectric are provided as microelectromechanical systems (MEMS) devices.

The present disclosure provides a method of determining the initial and subsequent motion including orientation of a segment of interest of a patient's body comprising: providing the patient with a micro-sized hollow bead implant placed within the cortical bone of the segment of interest of the patient's body and a circuit in the implant having a battery to provide power to the circuit, at least one sensor contained within the implant comprising (1) an accelerometer to provide a sensor output in response to acceleration of the implant, (2) a gyroscope to provide a sensor output in response to positioning of the implant, or (3) a magnetoelectric element to provide a sensor output in response to positioning of the implant in a magnetic field, alone or in combination, a processor to generate a data output in response to the sensor output, and a transceiver to generate a signal in response to the data output when the circuit is supplied with power; providing a remote receiver unit for receiving the data signal generated by the transceiver outside of the patient's body, wherein the receiver unit receives the data signal from the transceiver to determine the movement and positioning of the implant; generating a data signal in response to the sensor output; receiving the data signal with the powering/receiving unit; and determining the initial and subsequent position and orientation of the segment of interest of the patient's body from the data signal.

The present disclosure provides a method of determining the initial and subsequent motion including orientation of a segment of interest of a patient's body comprising: providing the patient with a micro-sized hollow bead implant placed within the cortical bone of the segment of interest of the patient's body and a circuit in the implant having a inductor and regulator to provide power to the circuit, at least one sensor contained within the implant comprising (1) an accelerometer to provide a sensor output in response to acceleration of the implant, (2) a gyroscope to provide a sensor output in response to positioning of the implant, or (3) a magnetoelectric element to provide a sensor output in response to positioning of the implant in a magnetic field, alone or in combination, a processor to generate a data output in response to the sensor output, and a transceiver to generate a signal in response to the data output when the circuit is supplied with power; providing a remote powering/receiving unit for supplying energy to the inductor and receiving the data signal generated by the transceiver outside of the patient's body, wherein when power is supplied to the circuit by the powering/receiver unit the sensor provides a sensor output in response to acceleration or positioning of the implant, and the powering/receiver unit receives the data signal from the transceiver to determine the movement and positioning of the implant; supplying energy to the inductor to power the circuit with the powering/receiving unit so as to provide a first output in response to acceleration of the implant and a second output in response to positioning of the implant; generating a data signal in response to the first output and the second output; receiving the data signal with the powering/receiving unit; and determining the initial and subsequent position and orientation of a segment of interest of a patient's body from the data signal.

The biosensors of the present disclosure provide diagnostic information *in situ* following implantation. For example, one or more biosensors can respond to interrogation by a central control unit via a transceiver so that the external controller can collect the data from the accelerometer, gyroscope, and/or magnetometer. The biosensor's transceiver and the external receiving unit can communicate via radiofrequency signals, e.g. using standard wireless or Bluetooth communications or by custom designed data transfer protocols. In certain embodiments, the biosensors can engage in one-way or two-way wireless communications with an external control unit, with each other or with other sensors, such as sensors on surgical instruments or probes.

Preferably the circuits in the device can be powered by systems external to the body, however internal power elements such as batteries are possible. External powering systems include generators of electromagnetic radiation to provide a current in the inductor. An internal passive power element, such as an inductance coil and a regulator, can be incorporated into the circuit so as to supply a constant voltage to the gyroscope, accelerometer, and/or magnetometer, and also the processor when irradiated with the electromagnetic radiation by the powering system. External interrogation and powering systems can be provided separately or as a single unitary apparatus. Some examples of interrogation and powering systems are described in U.S. Pat. No. 6,667,725 to Simons et al., U.S. Pat. No. 6,206,835 to Spillman et al., and U.S. Pat. No. 6,447,448 to Ishikawa et al., hereby mentioned.

### Brief Description of the Drawings

The invention may be understood by reference to the following description taken in conjunction with the accompanying drawings, in which, like reference numerals identify like elements, and in which:
FIG. 1 is a schematic, perspective external view of a microbead sensor according to the invention;
FIG. 1A is a schematic illustration of a microbead biosensor implanted into a cavity formed in a bone segment;
FIG. 2 is a schematic diagram of the internal components of an illustrative microbead biosensor;
FIG. 3 is a more detailed schematic block diagram of the biosensor of FIG. 2;
FIG.4 is a schematic block diagram of a system comprising multiple implanted biosensors and an external controller;
FIG 5 is schematic perspective illustration of a device for drilling bone and implanting biosensors according to the invention;
FIG 5A is a cross-section end view of the device of FIG. 5, showing the trocar lumen, drilling cartridge and biosensor implantation cartridge;
FIGS. 6A - 6E illustrate a process of implanting a microbead sensor according to the invention; FIG. 6A illustrates a step of puncturing a subject's tissue with a trocar-like instrument; FIG. 6B illustrates a step of drilling into cortical bone; FIG. 6C illustrates the resulting cavity formed in the subject's bone; FIG. 6D illustrates the insertion of a microbead biosensor into the cavity; and FIG. 6E illustrated the sensor after implantation;
FIGS 7A and 7B schematically illustrate the operation of a drilling cartridge according the invention. FIG. 7A illustrates the cartridge, in which the drill shaft and drill tip are stowed; FIG. 7B illustrates deployment of the drill tip at the distal end of a trocar;
FIG. 8 schematically illustrates the operation of the biosensor implantation cartridge;
FIG. 8A is a cross sectional view of both a drill cartridge and biosensor cartridge in an instrument, such as the device of FIG. 5, in which the drill cartridge is aligned with the trocar lumen; FIG. 8B shows the biosensor cartridge is aligned with the trocar lumen and FIG. 8C illustrates the loading of microbead biosensors into the biosensor cartridge;
FIG. 9A is another illustration of instrument according to the invention;
FIG. 9B is a cross-sectional view of the instrument of FIG. 9A;
FIG. 10 is a schematic illustration of the placement of three biosensors in different locations in a subject's leg using a trocar/needle like system. In this embodiment, the invention used for kinematic tracking of the femur, tibia and patella.
FIG. 11 is a schematic representation of a subject's femur, tibia and patella bones illustrating the kinematic data that can be derived by implanted biosensors according to the invention.

### Detailed Description

The term "magnetometer" as used herein refers to any device which can measure the direction and/or the intensity of a magnetic field in which the magnetometer is placed, whether using the earth's natural field or an artificially created field for positioning. Generally such devices are magnetoelectric. Magnetometers providing three component magnetic strength and direction measurements are included; however any magnetometer is encompassed by the term. In some embodiments a micro-sized hollow bead implant having one or more magnets contained within the implant can be used in conjunction with an external magnetometer to determine the orientation of the implant. Alternatively, in other embodiments a micro-sized hollow bead implant having a magnetoelectric element as the magnetometer is contained within the implant to provide a sensor output, such that the magnetoelectric element can be utilized to determine the positioning of the implant when it is in a magnetic field. In some embodiments the magnetometer is a graphene micro-electro mechanical systems (MEMS) or a nano-electro mechanical systems (NEMS) magnetometer.

The term "gyroscope" as used herein refers to any devices which can be used for maintaining orientation and/or angular velocity of the device. The most basic embodiment consists of a wheel mounted on three gimbals. In some embodiments a vibrating structure gyroscope is used to provide sensor output. In some embodiments the gyroscope is a graphene micro-electro mechanical systems (MEMS) or a nano-electro mechanical systems (NEMS) gyroscope.

The term "accelerometer" as used herein refers to any devices which can be used for measuring the proper acceleration of the device. In some embodiments the accelerometer is a graphene micro-electro mechanical systems (MEMS) or a nano-electro mechanical systems (NEMS) accelerometer.

In one embodiment, the present disclosure provides a micro-sized hollow bead implant for the body having an attached miniature gyroscope, accelerometer, and/or magnetometer used in combination or separately. Therefore, the present disclosure provides a device for a micro-sized hollow bead implant comprising: an external shell; a circuit in the implant having a power element to provide power to the circuit, and at least one sensor contained within the implant comprising (1) an accelerometer to provide a sensor output in response to acceleration of the implant, (2) a gyroscope to provide a sensor output in response to positioning of the implant, or (3) a magnetoelectric element to provide a sensor output in response to positioning of the implant in a magnetic field, alone or in combination, and a processor to generate a data output in response to the sensor output. An embodiment, having an accelerometer, gyroscope, and magnetoelectric element is illustrated in the accompanying drawings and described further below.

Alternatively, the present disclosure provides a device for micro-sized hollow bead implant comprising: an external shell; and at least one sensor contained within the implant comprising (1) an accelerometer to provide a sensor output in response to acceleration of the implant, (2) a gyroscope to provide a sensor output in response to positioning of the implant, or (3) a magnet to provide a sensor output to a magnetometer in response to positioning of the implant, alone or in combination, and a processor to generate a data output in response to the change in sensor output.

Kinematic tracking of a body part is an essential part many parts of clinical and sports medicine. In navigational surgery, navigational devices for the purpose of tracking movements would useful for facilitating surgical procedures and monitoring the outcome of prosthetic implant placement. Relating to magnetic fields, the prosthetic implant material can be enough of a marker for the magnetic field or there can be localizers which serve as markers in non-magnetic implants. The disclosure has markers on or in it for determining the position in a magnetic field. In physiotherapy, navigational devices for the purpose of tracking movements would be useful for understanding the physiological and biomechanical state of the patient. Relating to magnetic fields, an artificial magnetic field can be used as reference for magnetometer in the disclosure. In sports medicine, navigational devices for the purpose of tracking movements would be useful for understanding the physiological and biomechanical state of the athlete.

The miniature gyroscope, accelerometer, and/or magnetometer are placed inside of a bone segment and/or a joint prosthesis. In one preferred embodiment the implant can be implanted by press-fitting the device in the drill hole . The bore can be created after preliminary forging. The sensors can be sealed by a variety of means so as not to damage the equipment, e.g., with a sealant.

The term "micro" as used herein is intended to encompassed devices having at least one dimension that is less than a millimeter, preferably less than 100 micrometers or less than 10 micrometers and includes smaller devices, e.g. devices less than a micrometer or nanometer-sized structures.

The term "trocar" as used herein is intended to encompass any instrument capable of piercing tissue and serving as a conduit for insertion of other instruments through an inner lumen. "Trocar" encompasses needles and cannulas and other hollow or tubular tissue-piercing constructions, used with or without a separate obturator.

The term "drill" as used herein is intended to encompass any instrument capable of forming a cavity in a bone segment, including, for example, reamers, burrs and drill bits attached to shaft and powered by a rotatory motor.

The term "ovaloid," as applied to microbeads described herein is intended to encompass disc shaped, oval-shaped, hemispherical or otherwise rounded objects. Preferably, ovaloid microbeads have at least one surface (e.g., an equator) defined by a common radius from a center point, such that the microbead can easily slide through a cylindrical tube. In certain embodiments, the ovaloid can be defined by at least one convex surface that can mate with a rounded bottom of a drilled cavity during implantation, e.g., in a frictional or "press-fit" engagement. In certain embodiments the ovaloid microbead can have a spherical anterior surface and a generally flat posterior surface with a height (the greatest distance between the anterior and posterior surfaces) of less than 2 millimeters, preferably less than or equal to one millimeter and an equatorial diameter of less than or equal to 1 millimeter.

The term "battery" as used herein is intended to encompass any energy storage device including, for example, containers consisting of one or more cells, in which chemical energy is converted into electricity and used as a source of power. The term "battery" is also intended to encompass capacitive storage devices that store potential energy in the form of a electrostatic field and release the electric energy upon demand to device circuitry.

The term "energy harvesting device" as used herein is intended to encompass any device capable of converting kinetic energy into electrical energy that can power a device. For example, movement of magnet in an electromagnetic field can produce electricity from repetitive motion, e.g., walking. See, for example, US Patent Application Pub. No. US2017/0196507, herein mentioned.

The term "processor" as used herein is intended to encompass any device that performs operations on information put into it. The term "processor" also encompasses any logical circuitry that responses to and processes instructions and data.

The term "memory" as used herein is intended to encompass any physical device capable of storing information temporarily, like RAM (random access memory), or permanently, like ROM (read-only memory).

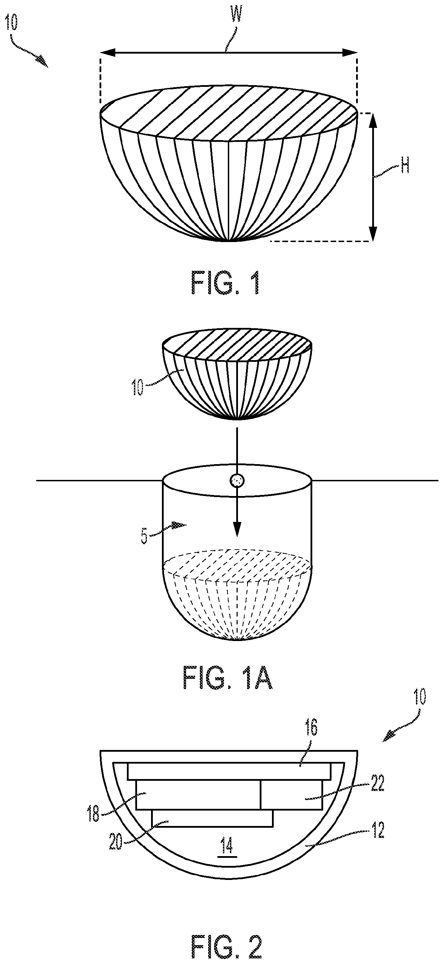

In FIG. 1 a schematic, perspective external view of a microbead biosensor **10** according to the invention is provided. The device can have a generally ovaloid shape. As shown, the biosensor **10** has a convex anterior surface and a flat posterior surface, i.e., a hemispherical shape. Other disc-like or even spherical shapes can also be devised. As shown, biosensor **10** has a diameter, **w,** of roughly 1 millimeter and a height, **h,** of roughly 0.7 millimeters.

FIG. 1A is a schematic illustration of a microbead biosensor **10** implanted into a cavity **5** formed in a bone segment **6.** The convex anterior surface of the biosensor **10** can be designed to match the shape of the bottom of cavity **5.**

In FIG. 2 a schematic diagram of the internal components of an illustrative microbead biosensor **10** is presented. External shell **12** defines a hollow interior chamber **14** housing, for example, a transceiver **16,** processor **18,** navigation sensor(s) **20** and power supply **22.**

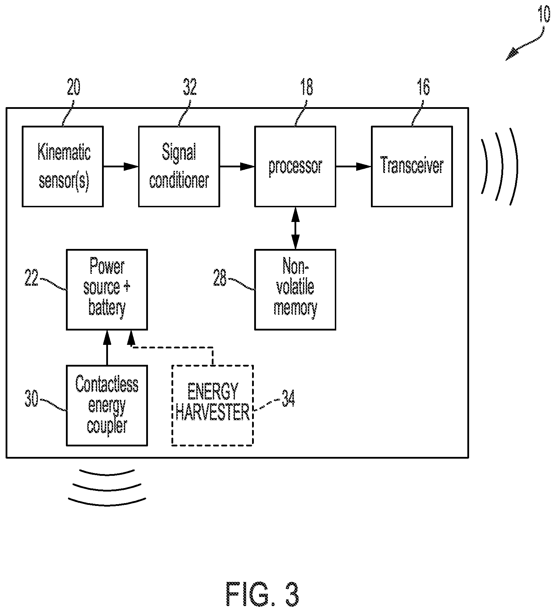

FIG. 3 is a more detailed schematic block diagram of the biosensor **10** comprising the transceiver **16,** processor **18** and kinematic sensor(s) **20.** The processor can further include a memory component **28,** e.g., a non-volatile memory chip. The sensor **10** can also include a signal conditioner **32** that pre-processes the output of the sensor(s) **20.** The power supply **22,** e.g., a battery, can be augmented by a contactless energy coupler **30** and/or an internal energy harvester **34.**

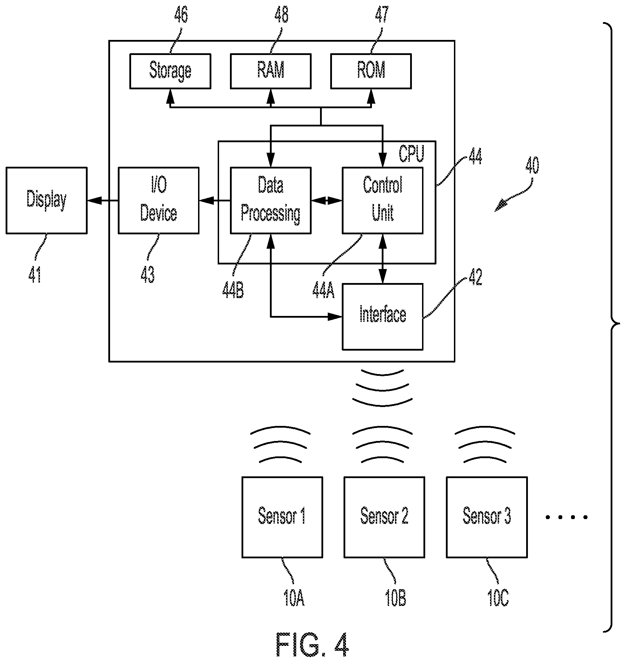

FIG.4 is a schematic block diagram of a system comprising multiple implanted biosensors **10A, 10B,** and **10C** an external controller (data analyzer module) **40** and display **41.** The controller **40** can comprise a data transceiver **42,** a central processing unit **44** (including for example control circuitry **44A** and a data processor **44B**), data storage **46,** and memory stacks of either read only memory (ROM) **47,** random access memory (RAM) **48** or both. The external controller can further comprise an interface **42** for energy and/or data transfer and an input/output device **49** for transferring display signals to the display **41.** Additionally, the system can include one or more sensors associated with a surgical instrument. For example, one or more of sensors **10A, 10B** or **10C** can be disposed on a surgical drill, cutter or the like so that the progress of surgery can be monitored or controlled before the implantation of one or more sensors into the patient's bone(s), as described further below.

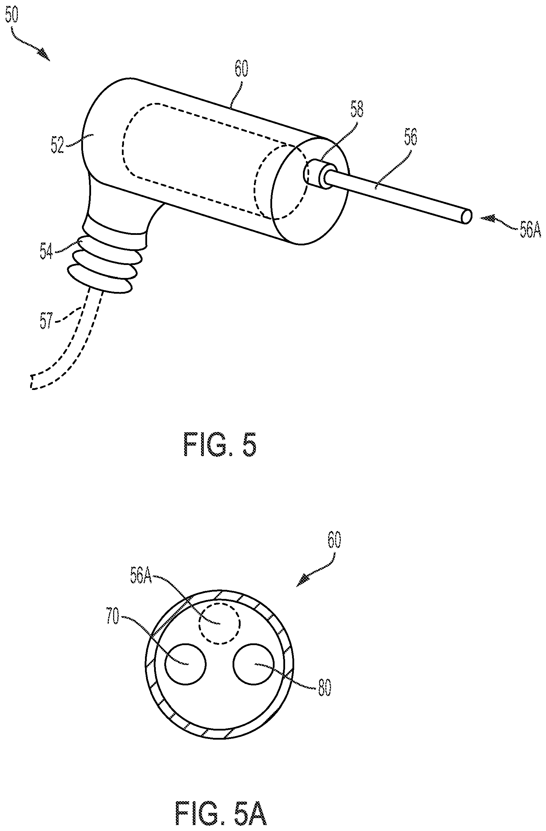

FIG 5 is schematic perspective illustration of instrument **50** for drilling bone and implanting biosensors according to the invention. Instrument 50 can include an instrument body **52,** handle **54,** and a trocar **56,** e.g., a detachable and replaceable trocar, having an inner lumen **56A** for passage of the drilling and implantation devices as discussed further below. Trocar **56** can be coupled to the instrument body **52** by coupler **58.** Optional cord **57** can supply electrical power and/or pneumatic pressure to the instrument **50.** Within the instrument body **52** a revolver barrel **60** is disposed.

As shown in FIG 5A, the revolver barrel **60** provides a housing for a drill cartridge **70** and a biosensor implantation cartridge **80.** The revolver barrel **60** can be rotated about a longitudinal axis so that either the drill cartridge **70** or the implantation cartridge **80** is aligned with the trocar lumen **56A.**

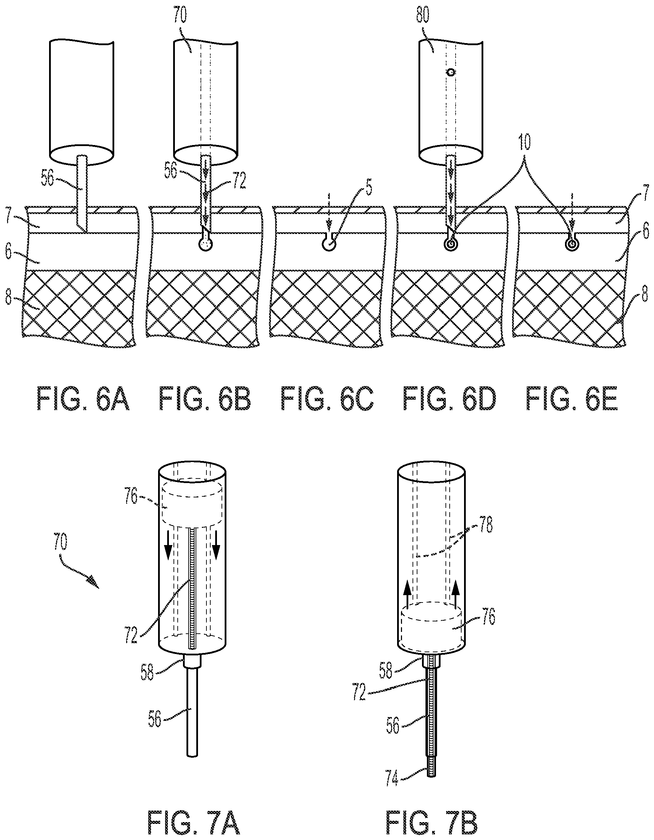

FIGS. 6A - 6E illustrate a process of implanting a microbead sensor **10** using a device according to the invention; FIG. 6A illustrates a step of puncturing a subject's tissue **7** with a trocar-like instrument **56.** FIG. 6B illustrates a step of actuating the drill cartridge **70** so that drill **72** can be deployed to drill into cortical bone **6;** FIG. 6C illustrates the resulting cavity formed in the subject's bone. FIG. 6D illustrates the deployment of the implantation cartridge **80** for insertion of a microbead biosensor **10** into the cavity. Finally, FIG. 6E illustrated the sensor **10** after implantation. Preferably the biosensors of the present invention are implanted into cortical bone **6** rather than cancellous bone **8** to ensure better fixation.

FIGS 7A and 7B schematically illustrate the operation of a drilling cartridge **70** according the invention. As noted above, drill cartridge **70** can be disposed in revolver barrel **60.** FIG. 7A illustrates the cartridge **70** aligned with the lumen of trocar **56** in a stowed condition with the drill shaft **72** and drill tip **74** within the cartridge body. FIG. 7B illustrates deployment of the drill shaft **72** deployed into the trocar **56** with the drill tip **74** at the distal end of a trocar **56** to drill a cavity in the target bone segment. Coupler **58** ensures coupling with the trocar lumen. Once the drill is deployed adjacent to a target site in a bone segment, motor **76** can be activated to rotate the drill tip **74** and form a cavity in the bone segment. Rails **78** can guide movement of the drill and motor up and down with the cartridge body.

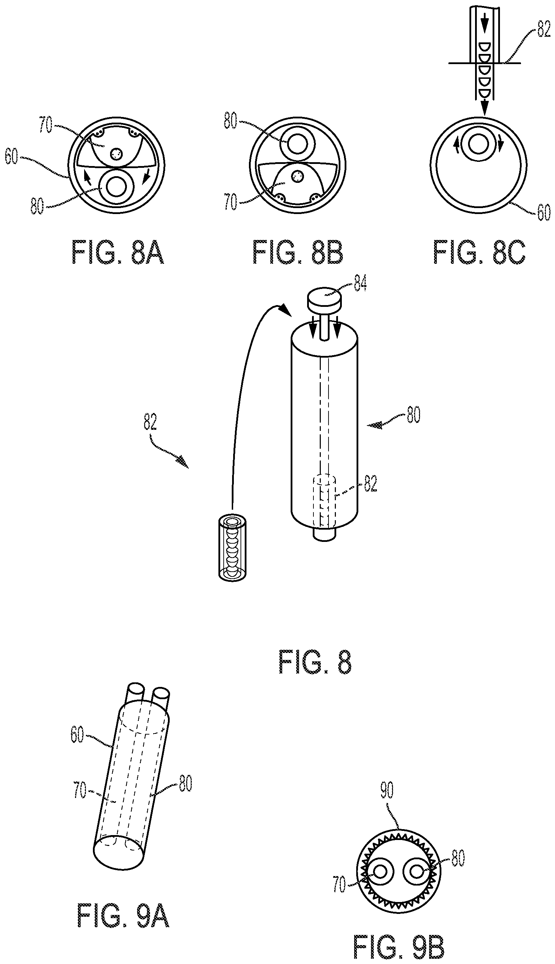

FIG. 8 schematically illustrates the operation of the biosensor implantation cartridge **80;** The implantation cartridge **80** can comprise a cradle **82** for multiple microbead sensors and a plunger or punch **84** for driving the microbeads into the bone cavities formed by the drill cartridge.

FIG. 8A is a cross sectional view of both a drill cartridge **70** and biosensor implantation cartridge **80** in an instrument **60,** such as the device of FIG. 5, in which the drill cartridge **70** is aligned with the trocar lumen; FIG. 8B shows the cartridges have been rotated 180 degrees such that the biosensor cartridge **80** is aligned with the trocar lumen and FIG. 8C illustrates the loading of microbead biosensors cradle **82** into the biosensor cartridge **80.**

FIG. 9A is another illustration of instrument according to the invention, in which a revolver barrel **60** can be rotated about a longitudinal axis so that either the drill cartridge **70** or the implantation cartridge **80** is aligned with the trocar **56.** FIG. 9B is a cross-sectional view of the instrument of FIG. 9A showing one mechanism for selection of which cartridge to be deployed. Cartridges **70** and **80** can include gear teeth on their external surfaces that mesh with a gears on the internal surface of the housing **60.** Ring **90** can be twisted to cause an inner portion of the housing to rotate relative to a outer portion, thereby permitting one or the other cartridge **70, 80** to be aligned with the trocar lumen.

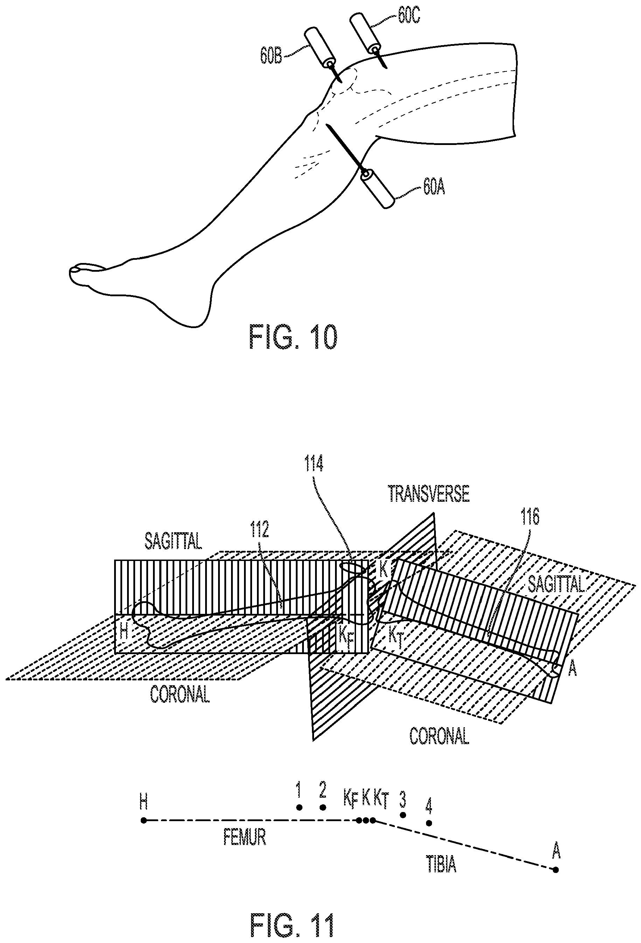

FIG. 10 is a schematic illustration of the placement of three biosensors in different locations in a subject's leg. Sensors are placed near the lateral femoral condyle or lateral cortex **112 ,** into the patella **114,** and near the tibial tuberosity **116.**

FIG. 11 is a schematic representation of a subject's femur, tibia and patella bones illustrating the kinematic data that can be derived by implanted biosensors according to the invention. Ideally, the system tracks kinematic output data for each sensor (**60A, 60B,** and **60C** of FIG. 10) that defines its position in 3D space, the distances between sensors, any movement in 3D of one sensor relative to another sensor (or relative to a fixed frame of reference), any acceleration in 3D of one sensor relative to another sensor (or relative to a fixed frame of reference), and any rotation in 3D of one sensor relative to another sensor (or relative to a fixed frame of reference). This data can be communicated directly to an external controller or received and stored (recorded) by an intermediate device (such as an ankle bracelet transceiver).

For the system illustrated in FIG. 11 the kinematic outputs can include (1) Range of motion (flexion/ extension in the sagittal plane), (2) varus or valgus from maximum extension to minimum of flexion (medio-lateral movements in the coronal plane), (3) femoro-tibial rotation (femoral coronal plane with respect to tibial coronal plane), (4) antero-posterior femoro-tibial gliding/sliding movements at any degree of extension or flexion (e.g., movement of knee marker **[Kf]** -- femoral center in the femoral coordinate's frame with respect to knee marker **[Kt]** - tibial center in the tibial coordinate's frame) and (5) patella antero-posterior and latero-medial movements along the knee flexion with respect to femoral and tibial frames.

FIG. 11 shows the femur **112,** the tibia **116** and the patella **114** bones. **H** is the center of the femoral head, **K** is the center of the knee with its projection on the distal femur **Kf** (center of the knee projected on the femur) and same for the proximal tibia **Kt.** Finally the center of the ankle (**A**). Between **H** and **Kf** (**K** with the knee in extension at rest) is the femoral mechanical axis and between **Kt** (**K** with the knee in extension at rest) and A is the tibial mechanical axis.

Three planes going through each axis are shown in FIG. 11. The coronal plane is defined thanks to anatomical landmarks on the distal femur (Trans epicondylar line TEA) and on the distal tibia (Transmalleolar line TMA). The sagittal and transverse planes are 90 degrees to each of the other planes.

If one considers only the sagittal plane in a simplified model we can draw two lines joined in the middle by a "hinge". The numbers **1, 2, 3** and **4** represent sensors affixed in each bone. (In this figure, there is no sensor implanted in the patella to simply the illustration.)

The present disclosure permits one to know the position of each sensor, e.g., with respect to **H, K** and **A.** For example, the system can track the position of **1** and **2** with respect to **3** and **4** from full extension to maximum flexion. Additionally, one can deduce (compute) the location of hip center H by hip circumduction movement, i.e., the rotation of sensors 1 and 2 in 3D. References **Kf** and **Kt** are constructs as **K** is a hinge, but micromovements between **Kf** and **Kt** provide valuable data. These movements can include antero-posterior (AP) in the transverse plane, and also medio-lateral (ML) also in the transverse plane and finally there are also potential displacement the sagittal and coronal planes combined (i.e. **Kf** and **Kt** move away from each other).

Those skilled-in-the-art, in light of the present disclosure, will appreciate that changes can be made in the specific embodiments which are disclosed herein and still obtain alike or similar results without departing from or exceeding the spirit or scope of the disclosure. The skilled artisan will further understand that any properties reported herein represent properties that are routinely measured and can be obtained by multiple different methods. The methods described herein represent one approach and other methods may be utilized without exceeding the scope of the present disclosure.

Within this specification, embodiments have been described in a way that enables a clear and concise specification to be written, but it is intended and will be appreciated that embodiments may be variously combined or separated. Every claimed feature should be deemed capable of multiple dependencies from other claimed features even if only one dependency is recited unless the combination of features is physically impossible.

The foregoing description of various forms of the invention has been presented for purposes of illustration and description. It is not intended to be exhaustive or to limit the invention to the precise forms disclosed. Numerous modifications or variations are possible in light of the above teachings. The forms discussed were chosen and described to provide the best illustration of the principles of the invention and its practical application to thereby enable one of ordinary skill in the art to utilize the invention in various forms and with various modifications as are suited to the particular use contemplated. All such modifications and variations are within the scope of the invention as determined by the appended claims.

## Claims

1. A device (50) for implantation of a biosensor (10) into a bone segment (6) comprising:
a hollow trocar (56) for accessing a subject's bone, the trocar having a sharp tip for piercing tissue such that a distal tip segment of the trocar can be positioned adjacent to a target bone location,
a drill cartridge (70) for forming a cavity in a bone, the drill cartridge (70) configured to facilitate passage of a drill through a lumen (56A) of the trocar to form a cavity (5) in the bone segment at the target location, **characterized in that** the device further comprises
a biosensor cartridge (80) configured for passage of the biosensor (10) through the lumen (56A) of the trocar to implant the biosensor (10) within the cavity formed by the drill, and
a revolver barrel (60) housing the drill cartridge (70) and the biosensor cartridge (80), the revolver barrel (60) being rotatable about a longitudinal axis to alternately align the drill cartridge (70) or the biosensor cartridge (80) with the lumen (56A) of the trocar.

2. The device of claim 1, wherein the trocar is releasably couplable to an instrument body (52) and, optionally, also includes a Luer-lock type coupler for connecting the trocar to the instrument body.

3. The device of any one of the preceding claims, wherein the drill cartridge further comprises a drill actuator for the drill.

4. The device of any one of the preceding claims, wherein the drill cartridge comprises a rotatable shaft (72) with a drill tip (74) disposed at its distal end.

5. The device of claim 4, wherein the actuator further comprises a drive coupler for coupling the rotatable shaft to a rotary motor.

6. The device of claim 1, wherein the biosensor cartridge further comprises a cylindrical chamber for storing at least one biosensor prior to implantation.

7. The device of claim 6, wherein the biosensor cartridge further comprises an implantation actuator, and wherein optionally the implantation actuator comprises a piston.

8. The device of claim 7, wherein the implantation actuator further comprises a pneumatic coupler for coupling the piston to a pneumatic pressure source.

9. The device of claim 1, wherein the device further comprises a stop for limiting the penetration of the drill, biosensor, or both into the bone segment.

10. The device of any one of the preceding claims, further comprising:
the biosensor, wherein the biosensor is a microbead biosensor for implantation into the cavity of the bone segment, the biosensor comprising:
a shell (12) for enclosing a sensor and configured for passage through the lumen of the trocar for implantation into the cavity of the bone segment, and
at least one kinematic sensor (20) disposed within the shell

11. The device of claim 10, wherein the kinematic sensor further comprises at least one sensor from the group of accelerometers, gyroscopes and magnetic sensors.

12. The device of claim 10, wherein the biosensor further comprises a power source (22) and, optionally, an energy harvesting device (34) to recovery energy from movement of the bone segment, wherein optionally the power source comprises a battery or a contactless energy coupler (30).

## Patentansprüche

1. Vorrichtung (50) zur Implantation eines Biosensors (10) in ein Knochensegment (6), umfassend:
einen hohlen Trokar (56) für den Zugang zu einem Knochen eines Patienten, wobei der Trokar eine scharfe Spitze zum Durchstechen von Gewebe aufweist, so dass ein distales Spitzensegment des Trokars in der Nähe einer Zielknochenstelle positioniert werden kann,
eine Bohrpatrone (70) zum Bilden eines Hohlraums in einem Knochen, wobei die Bohrpatrone (70) so konfiguriert ist, dass sie den Durchgang eines Bohrers durch ein Lumen (56A) des Trokars erleichtert, um einen Hohlraum (5) in dem Knochensegment an der Zielstelle zu bilden, **dadurch gekennzeichnet, dass** die Vorrichtung ferner umfasst:
eine Biosensorpatrone (80), die für den Durchgang des Biosensors (10) durch das Lumen (56A) des Trokars konfiguriert ist, um den Biosensor (10) in den durch den Bohrer gebildeten Hohlraum zu implantieren, und
einen Revolverzylinder (60), der die Bohrpatrone (70) und die Biosensorpatrone (80) aufnimmt, wobei der Revolverzylinder (60) um eine Längsachse drehbar ist, um abwechselnd die Bohrpatrone (70) oder die Biosensorpatrone (60) auf das Lumen (56A) des Trokars auszurichten.

2. Vorrichtung nach Anspruch 1, wobei der Trokar lösbar mit einem Instrumentenkörper (52) koppelbar ist und optional auch eine Luer-Lock-Kupplung zum Verbinden des Trokars mit dem Instrumentenkörper umfasst.

3. Vorrichtung nach einem der vorhergehenden Ansprüche, wobei die Bohrpatrone ferner einen Bohreraktuator für den Bohrer umfasst.

4. Vorrichtung nach einem der vorhergehenden Ansprüche, wobei die Bohrpatrone einen drehbaren Schaft (72) mit einer an seinem distalen Ende angeordneten Bohrspitze (74) aufweist.

5. Vorrichtung nach Anspruch 4, wobei der Aktuator ferner eine Antriebskupplung zum Koppeln der drehbaren Welle mit einem Drehmotor umfasst.

6. Vorrichtung nach Anspruch 1, wobei die Biosensorpatrone ferner eine zylindrische Kammer zur Aufbewahrung mindestens eines Biosensors vor der Implantation umfasst.

7. Vorrichtung nach Anspruch 6, wobei die Biosensorkartusche ferner einen Implantationsaktuator umfasst, und wobei der Implantationsaktuator optional einen Kolben umfasst.

8. Vorrichtung nach Anspruch 7, wobei der Implantationsaktuator ferner einen pneumatischen Koppler zum Koppeln des Kolbens mit einer pneumatischen Druckquelle umfasst.

9. Vorrichtung nach Anspruch 1, wobei die Vorrichtung ferner einen Anschlag zur Begrenzung des Eindringens des Bohrers, des Biosensors oder beider in das Knochensegment umfasst.

10. Vorrichtung nach einem der vorangegangenen Ansprüche, die ferner Folgendes umfasst:
den Biosensor, wobei der Biosensor ein Mikrokügelchen-Biosensor zur Implantation in den Hohlraum des Knochensegments ist, wobei der Biosensor umfasst:
eine Hülle (12) zum Umschließen eines Sensors, die so gestaltet ist, dass sie durch das Lumen des Trokars zur Implantation in den Hohlraum des Knochensegments geführt werden kann, und
mindestens einen in der Hülle angeordneten kinematischen Sensor (20).

11. Vorrichtung nach Anspruch 10, wobei der kinematische Sensor außerdem mindestens einen Sensor aus der Gruppe der Beschleunigungssensoren, Gyroskope und Magnetsensoren umfasst.

12. Vorrichtung nach Anspruch 10, wobei der Biosensor ferner eine Energiequelle (22) und optional eine Energiegewinnungsvorrichtung (34) zur Rückgewinnung von Energie aus der Bewegung des Knochensegments umfasst, wobei die Energiequelle optional eine Batterie oder einen kontaktlosen Energiekoppler (30) umfasst.

## Revendications

1. Dispositif (50) d'implantation d'un biocapteur (10) dans un segment osseux (6) comprenant :
un trocart creux (56) pour accéder à l'os d'un sujet, le trocart ayant une pointe acérée pour percer un tissu de telle sorte qu'un segment de pointe distale du trocart peut être positionné à proximité adjacente d'un emplacement osseux cible,
une cartouche de foret (70) pour former une cavité dans un os, la cartouche de foret (70) étant configurée pour faciliter le passage d'un foret à travers une lumière (56A) du trocart pour former une cavité (5) dans le segment osseux à l'emplacement cible, **caractérisé en ce que** le dispositif comprend en outre
une cartouche de biocapteur (80) configurée pour le passage du biocapteur (10) à travers la lumière (56A) du trocart pour implanter le biocapteur (10) dans la cavité formée par le foret, et
un barillet (60) abritant la cartouche de foret (70) et la cartouche de biocapteur (80), le barillet (60) étant rotatif autour d'un axe longitudinal pour aligner alternativement la cartouche de foret (70) ou la cartouche de biocapteur (80) avec la lumière (56A) du trocart.

2. Dispositif selon la revendication 1, dans lequel le trocart est accouplable de manière amovible à un corps d'instrument (52) et, facultativement, comporte également un coupleur de type Luer-lock pour connecter le trocart au corps d'instrument.

3. Dispositif selon l'une quelconque des revendications précédentes, dans lequel la cartouche de foret comprend en outre un actionneur de foret pour le foret.

4. Dispositif selon l'une quelconque des revendications précédentes, dans lequel la cartouche de foret comprend un arbre rotatif (72) ayant une pointe de foret (74) disposée à son extrémité distale.

5. Dispositif selon la revendication 4, dans lequel l'actionneur comprend en outre un coupleur d'entraînement pour accoupler l'arbre rotatif à un moteur rotatif.

6. Dispositif selon la revendication 1, dans lequel la cartouche de biocapteur comprend en outre une chambre cylindrique pour stocker au moins un biocapteur avant l'implantation.

7. Dispositif selon la revendication 6, dans lequel la cartouche de biocapteur comprend en outre un actionneur d'implantation, et dans lequel facultativement l'actionneur d'implantation comprend un piston.

8. Dispositif selon la revendication 7, dans lequel l'actionneur d'implantation comprend en outre un coupleur pneumatique pour accoupler le piston à une source de pression pneumatique.

9. Dispositif selon la revendication 1, dans lequel le dispositif comprend en outre une butée pour limiter la pénétration du foret, du biocapteur, ou des deux dans le segment osseux.

10. Dispositif selon l'une quelconque des revendications précédentes, comprenant en outre :
le biocapteur, dans lequel le biocapteur est un biocapteur à microbilles destiné à être implanté dans la cavité du segment osseux, le biocapteur comprenant :
une coque (12) pour enfermer un capteur et configurée pour un passage à travers la lumière du trocart pour une implantation dans la cavité du segment osseux, et
au moins un capteur cinématique (20) disposé à l'intérieur de la coque.

11. Dispositif selon la revendication 10, dans lequel le capteur cinématique comprend en outre au moins un capteur parmi le groupe constitué d'accéléromètres, gyroscopes et capteurs magnétiques.

12. Dispositif selon la revendication 10, dans lequel le biocapteur comprend en outre une source d'alimentation (22) et, facultativement, un dispositif de collecte d'énergie (34) pour récupérer de l'énergie d'un mouvement du segment osseux, dans lequel facultativement la source d'alimentation comprend une batterie ou un coupleur d'énergie sans contact (30).
